# EUROPÄISCHE PATENTSCHRIFT

(11) **EP 1 475 052 B1**
(45) Veröffentlichungstag und Bekanntmachung des Hinweises auf die Patenterteilung: **13.02.2008**
(21) Anmeldenummer: 04003507.3
(22) Anmeldetag: 17.02.2004
(51) Int. Cl.: A61C 13/15

(54) **Lichtpolymerisationsgerät mit Gebläse**
Photopolymerisation device with a fan
Appareil de photopolymérisation avec un ventilateur

(30) Priorität: 06.05.2003 DE 10320141
(43) Veröffentlichungstag der Anmeldung: 10.11.2004
(73) Patentinhaber: Firma Ivoclar Vivadent AG, FL-9494 Schaan (LI)
(72) Erfinder: Senn, Bruno, 9470 Buchs (CH); Plank, Wolfgang, 6830 Rankweil (AT)
(74) Vertreter: Baronetzky, Klaus

(56) Entgegenhaltungen:
- DE-A- 2 607 249
- FR-A- 2 629 999
- US-A- 4 298 806
- US-A- 5 634 711
- US-B1- 6 200 134

## Beschreibung

Die Erfindung betrifft ein Lichtpolymerisationsgerät, gemäß dem Oberbegriff von Anspruch 1.

Von Hand bedienbare Lichtpolymerisationsgeräte weisen üblicherweise eine recht starke Strahlungsquelle auf, die ihre Strahlung an eine Lichtleitstab abgibt und dazu dient, Dentalmaterialien zu polymerisieren. Die Strahlungsquelle ist in der Regel eine Lichtquelle, partiell aber auch eine Wärmequelle, wobei hier unter Licht auch ultraviolette Strahlung zu verstehen ist.

Derartige Lichtquellen und Stahlungsquellen geben regelmäßig Verlustwärme ab, die aus dem recht kompakten Handgerät abgeführt werden muss.

Zwar ist auch bereits vorgeschlagen worden, recht große Kühlrippen um die Lichtquelle herum vorzusehen und auf die natürliche Konvektion zu vertrauen, um die Wärmeabfuhr zu gewährleisten.

Gerade bei leistungsstarken Halogenlichtquellen ist eine derartige Kühlung jedoch nicht ausreichend, so dass in aller Regel eine Gebläsekühlung vorgenommen wird.

In aller Regel kommt hierbei ein Axialgebläse zum Einsatz, dessen Form dem Durchmesser des Gehäuses des Handgerätes angenähert ist, so dass der Kühlluftstrom im Wesentlichen parallel zur Längsachse des Handgerätes durch dieses hindurch verläuft.

Nachdem ein Anblasen des Behandlungsortes durch den Kühlluftstrom nicht akzeptabel wäre, ist der Luftauslass meist an der Rückseite des Gehäuses vorgesehen.

Um zu verhindern, dass der behandelnde Zahnarzt oder gegebenenfalls Zahntechniker unmittelbar angeblasen wird, ist es aus der DE-OS 34 11 996 bekannt geworden, die Luftaustrittsöffnung etwas nach schräg unten abzusenken, so dass der Kühlluftstrom zumindest nicht auf das Gesicht des Zahnarztes gerichtet ist.

Sämtliche bislang bekannten Lichtpolymerisationsgeräte mit Zwangskühlung erzeugen einen Luftstrom vom Patienten zum Zahnarzt. Dies bedeutet aber andererseits, dass für den Zahnarzt eine erhebliche Ansteckungsgefahr besteht; letztlich wird die Atemluft des Patienten dem Zahnarzt unmittelbar zugeleitet.

Die US 4,298,806 beschreibt ein Polymerisationsgerät, bei dem die Kühlung mit einem Luftstrom, der an der Gehäuserückseite einströmt, im Gehäuse am Ort des zu kühlenden Bauelementes umgelenkt wird und dann am unteren Ende des Handgriffes wieder ausströmt, erfolgen soll. Damit wird zwar eine Luftströmung vom Patienten zum behandelnden Zahnarzt vermieden, durch die Umlenkung sinkt der Wirkungsgrad jedoch deutlich und der Geräuschpegel steigt an.

Darüberhinaus beschreibt die US 6,200,134 B1 ein ähnliches Gerät, bei dem zusätzlich zur Kühlung des dort verwendeten LED-Arrays ein Kühlkörper vorgesehen ist, der wiederum über eine Zwangslüftung durch einen entlang der optischen Achse wirkenden Lüfter gekühlt werden soll.

Um die Ansteckungsgefahr etwas zu reduzieren, tragen manche Zahnärzte einen Mundschutz mindestens während der Zeit der Behandlung mit dem Lichtpolymerisationsgerät. Dies wird jedoch häufig als unangenehm empfunden, so dass diese Vorsichtsmaßnahme in manchen Fällen unterbleibt.

Ein weiteres Problem ist die Geräuschentwicklung des Handgerätes aufgrund des Gebläses. Für das Handgerät sind kompakte Abmessungen erwünscht. Dementsprechend verbleibt nur ein recht schmaler Spalt für die Bereitstellung des Gebläseluftstromes. Ein besonderes Problem ist der Reflektor für die Lichtquelle des Lichtpolymerisationsgerätes. Der Reflektor weist die an sich bekannte konische Form auf, und sein Außendurchmesser ist nur um weniges geringer als der Innendurchmesser des Gehäuses.

An dieser Stelle steht daher nur ein schmaler Ringspalt zur Verfügung, der hohe Strömungsgeschwindigkeiten und damit eine vergleichsweise große Geräuschentwicklung bedingt. Außerdem muss ein recht starkes Gebläse eingesetzt werden, um trotz dieses Strömungswiderstandes eine ausreichende Kühlung zu bewirken. Diese Gebläse bedingt nicht nur eine schnellere Entladung des Akkumulators, sondern auch eine recht starke Geräuschentwicklung.

Um dennoch eine vernünftige Bearbeitungszeit zu ermöglichen, ist es aus der DE-OS 42 11 230 bekannt, einen recht großen Akkusatz zu verwenden, der zudem den Schwerpunkt des Handgerätes handfreundlich nach unten absenkt und in den Pistolengriff des Handgerätes aufgenommen ist.

Zwar lässt sich durch diese Maßnahme der Nachteil der übrigen Lichtpolymerisation-Handgeräte, nämlich die verkürzte Akkulaufzeit reduzieren. Das Handgerät wird jedoch recht schwer und damit weniger handlich.

Ein weiteres Problem der bekannten Lichtpolymerisationsgeräte, das mit den vorstehend genannten Problemen verkettet ist, liegt in dem vergleichsweise geringen Kühlwirkungsgrad. Während die Kühlluft durch den Ringspalt um den Reflektor herum, also an dessen Außenrand, eine recht hohe Strömungsgeschwindigkeit aufweist, ist die Strömungsgeschwindigkeit und damit die Kühlwirkung ausgerechnet im Bereich der höchsten Temperatur, nämlich nahe der Glühwendel, ausgesprochen gering.

Um diese nachteilige Wirkung zu kompensieren, sind zwei Maßnahmen vorgeschlagen wurden: zum einen hat man Kühlrippen für den Kühlkörper über den gesamten Reflektor sich erstrecken lassen, so dass sie sogar bis in den Ringspalt reichen. Diese Lösung verbessert zwar den Kühlwirkungsgrad deutlich, erfordert jedoch die Herstellung eines aufwendig zu fertigenden und vergleichsweise schweren Reflektors.

Die andere Maßnahme sieht vor, den Strömungsquerschnitt im Bereich der Glühwendel, also am rückwärtigen Ende des Refklektors ebenfalls zu reduzieren. Hierdurch ist die Strömungsgeschwindigkeit auch dort vergleichsweise hoch, und es ist eine verbesserte Kühlung möglich. Nachteilig bei dieser Lösung ist jedoch, dass der Strömungswiderstand insgesamt noch weiter ansteigt, so dass zwar der Kühlwirkungsgrad verbessert, der Strömungswiderstand jedoch erhöht ist.

Daher liegt der Erfindung die Aufgabe zu Grunde, ein Lichtpolymerisationsgerät gemäß dem Oberbegriff von Anspruch 1 zu schaffen, das die Ansteckungsgefahr für Zahnärzte dem Grunde nach reduziert, hinsichtlich der Handhabbarkeit aufgrund vergleichsweise geringen Akkugewichts verbessert ist, ein geringeres Geräuschniveau bietet, aber dennoch abgesenkte Herstellkosten ermöglicht.

Diese Aufgabe wird erfindungsgemäß durch Anspruch 1 gelöst. Vorteilhafte Weiterbildungen ergeben sich aus den Unteransprüchen.

Die erfindungsgemäße Lösung sieht vor, den Kühlluftstrom quer zum Gehäuse des Lichtpolymerisationsgerätes auszubilden. Hierbei sei unter Gehäuse das eigentliche Gehäuse, also ohne Handgriff zu verstehen.

Der Kühlluftstrom erstreckt sich bevorzugt genau an dem Sockel der Lichtquelle vorbei, also von einer Gehäuseseite zur anderen Gehäuseseite. Er ist deutlich kürzer als die Kühlluftströme der bislang bekannten Handgeräte, beispielsweise ein Viertel oder ein Drittel von deren Länge. Die Strömungsgeschwindigkeit kann drastisch abgesenkt werden, ebenfalls beispielsweise auf ein Drittel. Der Kühlkörper kann wesentlich leichter bauend ausgebildet sei, nachdem er den einzigen nennungswerten Strömungswiderstand bildet. Die Kühlung erfolgt unmittelbar an der Stelle, an der die Verlustwärme entsteht, also an der Lichtquelle.

Die Kühlrippen verlaufen in an sich bekannten Weise parallel zur Kühlungsstrom. Nachdem dieser jedoch um einen rechten Winkel gegenüber den bekannten Kühlluftströmen gedreht ist, erstrecken sich die Kühlrippen ebenfalls in einer um etwa einen rechten Winkel gedrehten Ausrichtung.

Erfindungsgemäß besonders günstig ist es, wenn eine intensive Wärmeleitverbindung zwischen der Lichtquelle und dem recht kleinen Kühlkörper vorgesehen ist.

Die intensive Wärmeleitverbindung kann entweder direkt vorgesehen sein, also so, dass die Lichtquelle unmittelbar mit dem Kühlkörper verbunden ist, oder sie kann indirekt vorgesehen sein. Bei dieser Ausgestaltung ist die Lichtquelle mit dem Sockel und/oder dem Reflektor in guter Wärmeleitverbindung, und der Sockel und/oder der Reflektor ist wiederum mit dem Kühlkörper verbunden. Es versteht sich, dass erfindungsgemäß dafür Sorge getragen ist, dass gerade bei der indirekten Wärmeleitverbindung der Wärmeübergangswiderstand je gering bleibt.

Bei Verwendung einer Halogenlichtquelle mit integriertem Reflektor ist es erfindungsgemäß günstig, wenn die Wärmeleitfähigkeit zwischen der Glühwendel und dem Kühlkörper möglichst gut ist. Hierzu kann beispielsweise der Sockel der Halogenlichtquelle in guter Wärmeleitverbindung mit dem Kühlkörper stehen. Auch ist es möglich, den Kühlkörper so zu dimensionieren, dass der Anschlussstutzen der Halogenlichtquelle in ihn eintritt. Der Wärmewiderstand kann dort durch Einbringung von Wärmeleitpaste noch weiter verbessert werden.

Ein weiterer besonders günstiger Gesichtspunkt ist, dass der recht kleine und vergleichsweise heiße Kühlkörper innenliegend, also mit recht großen Abstand zur Außenwand des Gehaüses hin, angeordnet sein kann.

Hierdurch lässt sich zum einen den Kühlwirkungsgrad deutlich verbessern, denn im Kühlluftstrom liegen dann ausgesprochen heiße Flächen. Zum anderen ist gewährleistet, dass sich das Gehäuse außen nicht warm anfühlt.

Besonders vorteilhaft ist es, wenn die Nenndrehzahl des oder der Gebläse weniger als 3000 U/min beträgt und insbesondere die Schallemission des Gebläses in Betrieb weniger als 25 dB beträgt.

Durch die intensive Kühlwirkung kann mit einer geringen Gebläsedrehzahl gearbeitet werden, aufgrund der geringeren Turbolenzen sinkt auch die Schallemission des Gebläses deutlich.

Der für das Gehäuse verwendete Kunststoff wird thermisch nicht belastet, nachdem er aufgrund des großen Abstandes eher kühl bleibt. Besonders günstig ist es auch, dass der rückwärtige Bereich des Gehäuses des Lichtpolymerisationsgerätes kalt verbleibt. An dieser Stelle sind häufig empfindliche elektronische Bauteile oder auch eine LCD-Anzeige vorgesehen, deren Betriebstemperatur recht beschränkt ist und die bei erhöhter Betriebstemperatur eine verminderte Lebensdauer aufweist. Auch der Akkusatz wird nicht von dem Kühlluftstrom beeinträchtigt, und es ist möglich, mit einem vergleichsweise kleine Akkumulator auszukommen, nachdem ein Gebläse mit geringer Leistung einsetzbar ist. Hierdurch sinkt auch die Schallemission durch das Gebläse, beispielsweise auf 20 dB.

Erfindungsgemäß besonders günstig ist es aber auch, dass der Wärmeübergang gezielt von der heißesten Stelle - und ausschließlich von der heißesten Stelle - zum Kühlkörper verlaufen kann. Dies bedeutet, dass keine Temperaturvergleichmäßigung innerhalb des Gehäuses stattfindet, die das Gehäuse insgesamt erwärmen würde und damit auch die Kühlwirkung durch den Kühlkörper herabsetzen würde.

Durch die Strömungsrichtung des Kühlluftstromes quer zur Längsrichtung des Handgerätes ist sichergestellt, dass der behandelnde Zahnarzt außerhalb des Kühlluftstromes bleibt. Die Ansaugung erfolgt auch nicht unmittelbar vom Patienten her sondern seitlich. Hierdurch ist sichergestellt, dass kein direkter Austausch vom Schwebeteilchen zwischen dem Patienten und dem Zahnarzt durch den Kühlluftstrom bereitgestellt wird. Die Gefahr von Ansteckungen sinkt damit erheblich, auch ohne dass der Zahnarzt einen Mundschutz trägt.

Der erfindungsgemäße Quer-Kühlungskanal kann in an sich bekannter Weise mit geeigneten Maßnahmen berührungsgeschützt ausgebildet sein. Beispielsweise können Lüftungsschlitze in dem Gehäuse vorgesehen sein, die mit den Einzel-Kühlluftkanälen zwischen den Rippen des Kühlkörpers fluchten.

Es ist auch möglich, ein Rost oder Gitter in die jeweilige Gehäusehälfte zu integrieren, um die Lufteintrittsöffnung beziehungsweise die Luftaustrittsöffnung zu bilden. Ein Rost kann auch abnehmbar ausgebildet sein, um die leichte Reinigung des Kühlkörpers oder gegebenenfalls des Gebläses von Staub zu ermöglichen.

Besonders vorteilhaft ist es, wenn das Gehäuse teilbar ist und insbesondere eine Betätigungsvorrichtung, insbesondere ein Druckknopf für ein Abnehmen eines Gehäuseteiles vorgesehen ist, welches Gehäuseteil den Kühlkörper und/oder das Gebläse freigibt. Durch die Abnahme des Gehäuseteils ist ein leichter Zugang zum Gebläse gewährleistet. Durch diese Maßnahme lässt sich das Gebläse zur Reinigungs- oder Wartungszwecken leicht entfernen.

In diesem Zusammenhang ist es besonders günstig, wenn die elektrischen Kontakte für den Gebläsemotor steckbar sind und beim Einstecken des Gebläses in die Arbeitsposition die betreffende Steckverbindung automatisch hergestellt wird. Diese Ausgestaltung vereinfacht weiter die Reinigung, Wartung und/oder den Austausch des Gebläses, wenn dies einmal erforderlich sein sollte.

Der Kühlkörper selbst schützt hierbei die feuchtigkeitsempfindliche Elektronik, so dass auch beispielsweise auch ein Abwischen der Kühlrippen mit einem angefeuchteten Lappen, einen Tupfer oder dergleichen gefahrlos möglich ist.

Der Kühlkörper kann einseitig oder beidseitig des Gebläses ausgebildet sein; bevorzugt ist er einseitig ausgebildet.

Erfindungsgemäß besonders günstig ist es ferner, wenn der Kühlkörper in engen räumlichen und zugleich thermischen Kontakt mit dem Sockel und/oder dem Anschlussstutzen der Lichtquelle ist. Beispielsweise kann der Kühlkörper diesen Bereich U-förmig umgreifen, um einen niedrigen Wärmeübergangwiderstand bereitzustellen.

Erfindungsgemäß besonders günstig ist es, wenn saugseitig des Gebläses ein freies Anströmen mit geringen Strömungswiderstand vorgesehen ist und druckseitig - gegebenenfalls nach einer räumlich knapp bemessenen Druckzone - die durch das Gebläse verwirbelte Kühlluft an den Kühlrippen entlang strömt und in den dort gebildeten Einzel-Strömungskanälen ausgerichtet und beruhigt wird. Sowohl der Strömungswirkungsgrad als auch der Kühlwirkungsgrad ist durch diese Lösung besonders gut.

Weitere Vorteile, Einzelheiten und Merkmale ergeben sich aus der nachstehenden Beschreibung mehrere Ausführungsbeispiele der Erfindung anhand der Zeichnungen.

Es zeigen:
- Fig. 1: eine Seitenansicht einer Ausführungsform eines erfindungsgemäßen Lichtpolymerisationsgerätes;
- Fig. 2: eine vergrößerte Ansicht der Ausführungsform gemäß Fig. 1;
- Fig. 3: eine teilweise aufgebrochene Ansicht, entsprechend der Ansicht gemäß Fig. 2;
- Fig. 4: eine ebenfalls aufgebrochene Ansicht der Ausführungsform gemäß den Fig. 1 bis 3, wobei die gegenüberliegende Seite gegenüber der Ansicht gemäß Fig. 1 bis 3 dargestellt.ist;
- Fig. 5: ein Schnitt durch die Ausführungsform gemäß den Fig. 1 bis 4, wobei eine vertikale Schnittrichtung gewählt ist;
- Fig. 6: ein Schnitt der Ausführungsform gemäß den Fig. 1 bis 5, wobei eine horizontale Schnittrichtung gewählt ist;
- Fig. 7: eine zweite Ausführungsform eines erfindungsgemäßen Lichtpolymerisationsgerätes, unter Darstellung eines wesentlichen Details;
- Fig.8: eine dritte Ausführungsform eines erfindungsgemäßen Lichtpolymerisationsgerätes, unter Darstellung eines wesentlichen Details; und
- Fig. 9: eine Darstellung einer vierten Ausführungsform eines erfindungsgemäßen Lichtpolymerisationsgerätes, wobei Kühlkörper und Lichtquelle dargestellt sind.

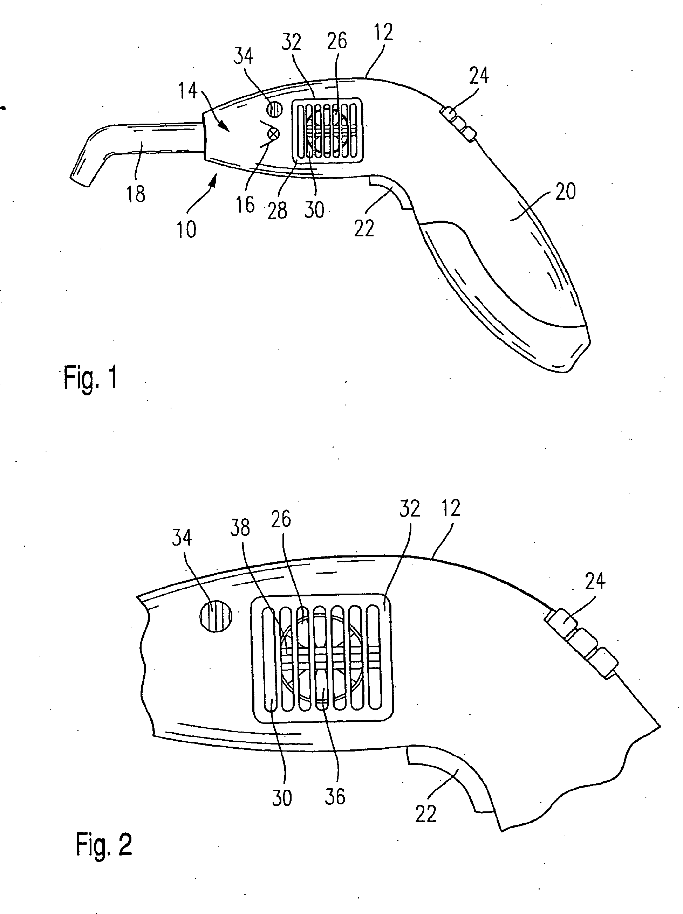

Das in Fig. 1 dargestellte Lichtpolymerisationsgerät 10 weist ein Gehäuse 12 auf, in dem eine schematisch dargestellte Lichtquelle 14 angeordnet ist. Die Lichtquelle, die mit einem Reflektor 16 versehen ist, sendet Polymerisationsstahlung nach vorne, in die Lichtaustrittsrichtung aus. Die Strahlung durchtritt einen Lichtleitstab 18, der in an sich bekannter Weise endseitig abgekröpft ist.

Das Lichtpolymerisationsgerät 10 weist einen im Wesentlichen pistolenförmigen Handlauf auf. Dementsprechend ist ein Handgriff 20 vorgesehen, und die Auslösung eines Lichtpolymerisationszyklusses erfolgt durch die Betätigung eines Schalters 22. Der Schalter 22 ist an dem Übergang zwischen Handgriff 20 und Gehäuse 12 vorgesehen. Dem Schalter 22 gegenüberliegend ist ein Programmwahlschalter 24 ausgebildet, der gegebenenfalls auch mit einem LCD-Display ausgestattet ist.

Erfindungsgemäß ist das Lichtpolymerisationsgerät mit einem Gebläse 26 ausgestattet, dessen Drehachse sich quer zur Lichtaustrittsrichtung der Lichtquelle 14 erstreckt. Das Gebläse 26 ist etwa mittig in dem Gehäuse 12 ohne den Handgriff 20 betrachtet angeordnet. Das Ansaugen und das Ausblasen des Gebläses 26 erfolgt seitlich. Aus Fig. 1 ist eine Luftaustrittsöffnung 28 ersichtlich, die über eine Mehrzahl von Luftschlitzen 30 verfügt .

In diesem Ausführungsbeispiel ist die Luftaustrittsöffnung 28 in einem separaten Gehäuseteil 32 ausgebildet, das als eine Art Rost vorgesehen ist. Durch Betätigung eines Druckknopfes 34 lässt sich das Gehäuseteil 32 entfernen, so dass das Gebläse und ein dort vorgesehener Kühlkörper frei zugänglich sind und leicht gereinigt werden können.

Das Gehäuse 12 - wieder betrachtet ohne den Handgriff 20 - ist wesentlich länger als breit. Hierdurch ergibt sich ein ausgesprochen kurzer Kühlluftstrom quer durch das Gehäuse hindurch, und auch die Gesamtbaulänge des Lichtpolymerisationsgerätes lässt sich verkürzen.

Aus Fig. 2 ist ersichtlich, dass das Gebläse 26 als Axialgebläse ausgebildet ist, das in an sich bekannter Weise eine Vielzahl, beispielsweise 3 bis 10, Flügel 36 aufweist.

In diesem Ausführungsbeispiel ist eine thermische Verbindung zwischen der Lichtquelle und einem Kühlkörper 40 über eine gedruckte Schaltung 38 realisiert.

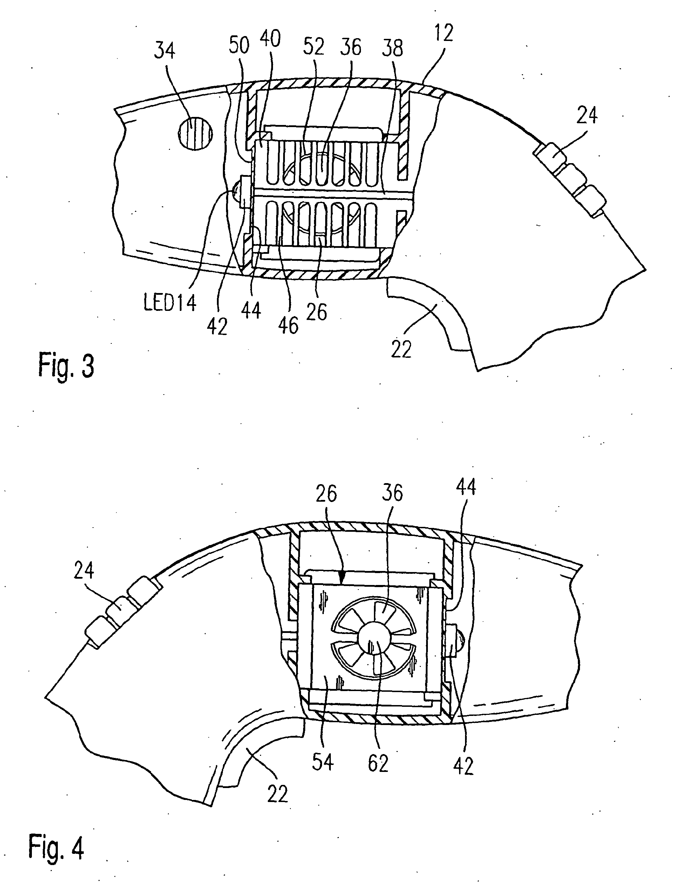

Dies ist in Fig. 3 besser ersichtlich.

Dementsprechend wird der Kühlkörper partiell durchtreten durch die Platine 38, die sich in Längsrichtung des Gehäuses erstreckt und Leiterbahnen für den Anschluss der Lichtquelle trägt. Die Lichtquelle selbst ist in einen Sockel 42 eingesteckt, der auf einer gedruckten Schaltung 44 angebracht ist. In den Sockel 42 ist in an sich bekannter Weise die wärmeabgebende Lichtquelle eingesteckt. Über deren Anschlussstifte wird die Wärme abgeführt und dem Kühlkörper 40 zugeleitet.

Der Kühlkörper 40 weist eine Vielzahl von benachbarten und zueinander parallelen Kühlrippen 46 auf. Die Kühlrippen erstrecken sich von der Längsmittenachse ausgehend nach oben und unten und sind seitlich so ausgerichtet, dass sie in seitlicher Richtung einen möglichst geringen Strömungswiderstand bieten.

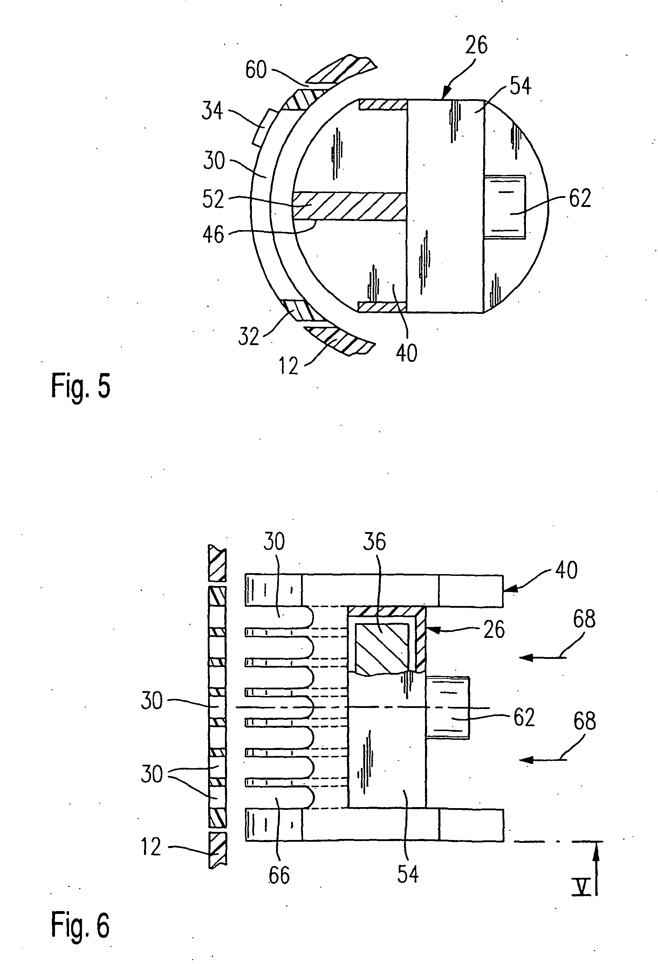

Der Kühlkörper ist in diesem Ausführungsbeispiel in besonderer Form ausgebildet, die aus Fig. 5 besser ersichtlich ist. Seine halbzylindrische Außenkontur nähert sich der Innenform des Gehäuses 12 an, ohne dass eine Berührung stattfindet. Die großflächige Ausgestaltung der Kühlrippen 46 erlaubt eine gute Wärmemitnahme trotz eines vergleichsweise kurzen Strömungsweges.

Auch wenn hier ein einstückiger Kühlkörper dargestellt ist, versteht es sich, dass anstelle dessen auch eine mehrstückige Ausgestaltung aus Gründen der leichteren Fertigung möglich ist, wobei dann auf einen geringen Wärmeübergangswiderstand zwischen den Teilen des Kühlkörpers geachtet werden muss

Wie aus Fig. 3 ersichtlich ist, ist ein vorderer Endbereich 50 des Kühlkörpers 40 mit einer größeren Materialstärke ausgebildet. An dieser Stelle wird die Wärme der Lichtquelle in den Kühlkörper eingeleitet, und die Vergrößerung der Materialstärke ermöglicht eine bessere Vergleichmäßigung der Wärmeeinleitung.

Die Kühlrippen 46 sind im Wesentlichen in gleicher Stärke ausgebildet und gleich voneinander beabstandet. Über einen Mittelsteg 52 sind sie miteinander verbunden, und zwar sowohl in mechanischer Hinsicht als auch hinsichtlich der Wärmeleitung.

Der Kühlkörper ist in an sich bekannter Weise aus einem gut wärmeleitenden Material wie Aluminium oder einer Aluminiumlegierung.

Zusammen mit dem Gebläse 26 bildet er eine kompakte Einheit, bei der, wie es aus Fig. 4 ersichtlich ist, ein Gebläsegehäuse 54 in seinen Abmessungen dem Kühlkörper 40 entspricht. Bevorzugt sind das Gebläsegehäuse 54 und der Kühlkörper 40 aneinander befestigt, so dass sie als kompakte Einheit vormontiert eingebaut werden können. Das Gebläse 26 ist um weniges von den Kühlrippen 40 beabstandet montiert.

In dem dargestellten Ausführungsbeispiel ist der Kühlkörper 40 druckseitig des Gebläses 46 vorgesehen, wobei es sich versteht, dass bei Bedarf auch eine Anordnung auf der Saugseite - oder gegebenenfalls eine Anordnung auf beiden Seiten - des Gebläses möglich ist.

Wie es aus Fig. 5 ersichtlich ist, folgt der Kühlkörper 40 der Kontur des Gehäuses 12, wobei ein deutlicher Spalt 60 vorgesehen ist, der auch der thermischen Trennung dient.

In an sich bekannter Weise weist das Gebläse 26 einen Gebläsemotor 62 auf. Auch wenn in Fig. 5 lediglich die linke Seite des Gehäuses 12 unter Darstellung des abnehmbaren Gehäuseteiles 32 dargestellt ist, versteht es sich, dass eine entsprechende Ausgestaltung mit Luftschlitzen entsprechend den Luftschlitzen 30 auch im rechten Bereich des Gehäuses vorgesehen ist.

Aus Fig. 6 ist ersichtlich, dass sich die Luftschlitze 30 ausgerichtet zu Einzel-Strömungskanälen 66 erstrecken. Die Kühlluftströmung durchtritt das Gehäuse 12 entsprechend der Richtung der Pfeile 68, wobei durch die erfindungsgemäße Ausgestaltung ein recht großer Strömungskanal ausgebildet wird.

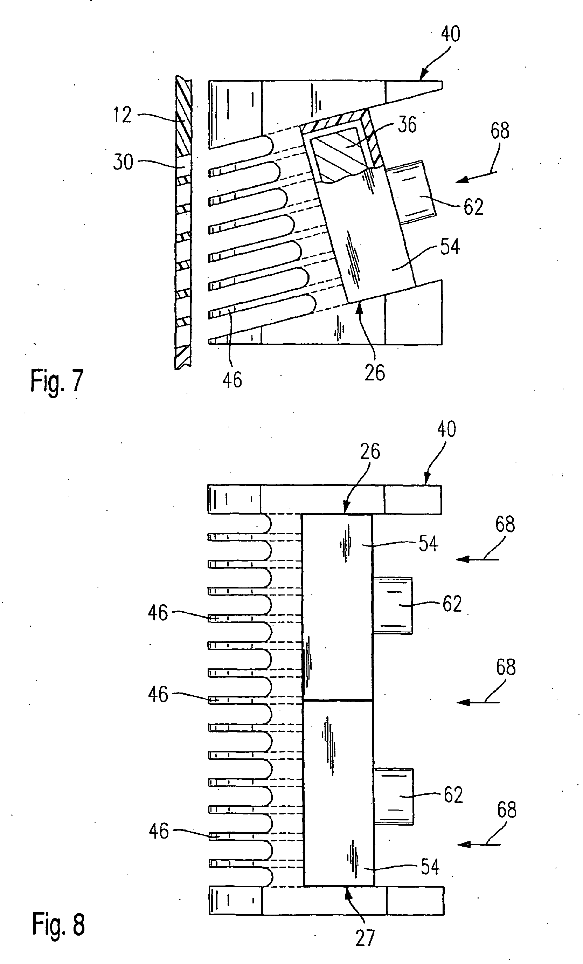

Aus Fig. 7 ist eine modifizierte Ausgestaltung eines erfindungsgemäßen Lichtpolymerisationsgerätes ersichtlich. Hier wie auch in den weiteren Figuren entsprechen gleiche Teile gleichen Bezugszeichen.

Die Strömungsrichtung des Strömungskanals 68 ist hier schräg ausgerichtet, etwa in einem Winkel von 105° zur Lichtaustrittsrichtung der Lichtquelle. Die Luftströmung der warmen Kühlluft ist daher etwas schräg nach hinten gerichtet, so dass sie noch stärker vom Patienten wegweist.

Es versteht sich, dass die Strömungsrichtung des Kühlluftkanals in beliebiger Weise an die Erfordernisse anpassbar ist.

Aus Fig. 8 ist eine dritte Ausführungsform des erfindungsgemäßen Lichtpolymerisationsgerätes ersichtlich. Bei dieser Ausgestaltung sind zwei Gebläse 26, 27 hintereinander, also in Längsrichtung des Gehäuses 12 oder parallel zur Lichtaustrittsrichtung hintereinander, angeordnet. Damit kann der Kühlluftstrom einen wesentlich vergrößerten Querschnitt einnehmen, und es lässt sich ein wesentlich größerer Kühlkörper 40 einsetzen.

Diese Lösung ist besonders geeignet, wenn Hochleistungs-Lichtquellen zum Einsatz gelangen, wobei durch den vergrößerten Kühlkörper das Gewicht des Handgerätes 10 zunimmt.

Erfindungsgemäß besonders günstig ist es, dass bei sämtlichen Ausführungsformen die Einheit aus Gebläse und Kühlkörper kompakt zusammengefügt und auch entnehmbar ist. Durch Betätigung des Druckknopfes (vgl. auch Fig. 5) lässt sich eine Verriegelungsmechanik lösen und die Einheit entnehmen. Auch ein Ersatz der oder des Gebläses je nach Erfordernis ist auf diese Art und Weise leicht möglich. Es ist auch möglich, die Einheit unter einer gewissen Federvorspannung zu lagern, wobei auch eine automatische Kontaktierung durch entsprechend geeigneter und gestalteter Kontaktflächen möglich ist.

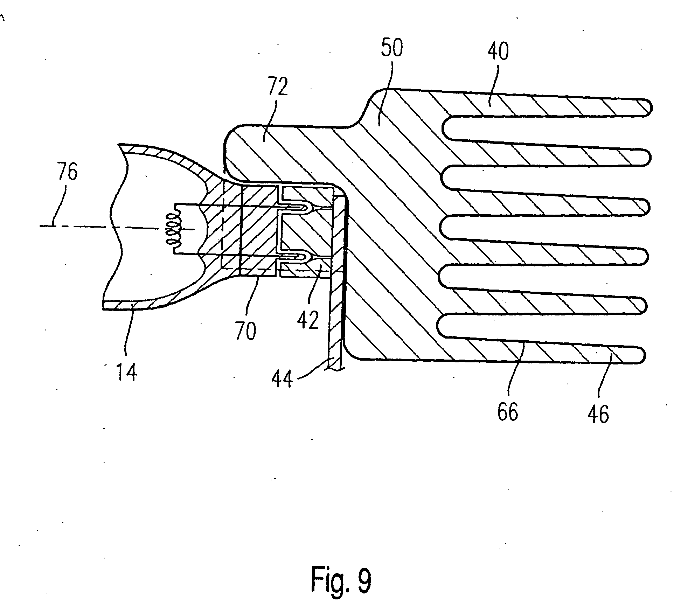

Auch die Ausführungsform gemäß Fig. 9, die sich durch einen intensiven Kontakt zwischen der Wärmequelle und dem Kühlkörper 40 auszeichnet, erlaubt eine Entnahme. Hierzu ist der den Sockel 42 und einen Anschlussstutzen 70 der Lichtquelle 14 umschließende Teil 72 des Kühlkörper 40 im Wesentlichen U-förmig ausgebildet.

Aus Fig. 9 ist auch die Lage der optischen Achse 76, die durch die Lichtaustrittsrichtung verläuft, der Lichtquelle 14 ersichtlich. Die optische Achse 76 erstreckt sich im Wesentlichen parallel zur Längsachse des Gehäuses, wenn der zylindrische Teil des Gehäuses, also das Gehäuse ohne Handgriff, betrachtet wird.

Aus dem U-förmigen Kühlkörper heraus erstreckt sich auch die gedruckte Schaltung 44, die in dem dargestellten Ausführungsbeispiel einseitig nach unten verläuft.

Es versteht sich, dass hier bei Bedarf auch elektronische Bauteile unterbringbar sind, wobei es jedoch bevorzugt ist, in diesem thermisch belasteten Bereich lediglich die Anschlussleitungen unterzubringen und die Elektronik für das Polymerisationsgerät 10 im rückwärtigen Bereich des Gehäuses 12, also etwa zwischen Schalter 22 und Programmwahlschalter 24, vorzusehen.

Auch bei dieser Ausführungsform erstrecken sich großflächig Kühlrippen 46, zwischen denen dementsprechend Einzel-Strömungskanäle 66 ausgebildet sind, ausgehend von einem vorderen Endbereich 50.

Bei dieser Ausführungsform erstrecken sich die Kühlrippen 46 in einer horizontalen Ebene.

Es versteht sich, dass die Ausgestaltung im Einzelnen in weiten Bereichen an die Erfordernisse anpassbar ist. Beispielsweise kann es zur Verbesserung der Wärmeleitung zwischen dem Sockel 42 und dem Teil 72 des Kühlkörper 40 vorgesehen sein, dort eine Wärmeleitpaste einzubringen. Auch versteht es sich, dass bei Bedarf auch eine elektrische Isolierung zwischen der gedruckten Schaltung 44 und dem Kühlkörper realisiert ist.

## Patentansprüche

1. Lichtpolymerisationsgerät, insbesondere zum Aushärten von lichtpolymerisierbaren Dentalmaterialien, mit einem Handgriff (20), einem Gehäuse und einem in das Gehäuse eingebauten Gebläse (26), insbesondere einem Axialgebläse, und einer Lichtquelle, die Polymerisationsstrahlung aussendet, **dadurch gekennzeichnet, dass** die Drehachse des Gebläses (26) sich sowohl quer zur optischen Achse (76) der Lichtquelle (14) als auch quer zur Längsachse des Handgriffs (20) erstreckt.

2. Lichtpolymerisationsgerät nach Anspruch 1, **dadurch gekennzeichnet, dass** die die Drehachse unter einem Winkel von 60° bis 90° zur optischen Achse (76) angeordnet ist.

3. Lichtpolymerisationsgerät nach einem der vorhergehenden Ansprüche, **dadurch gekennzeichnet, dass** der Winkel zwischen der Drehachse des Gebläses (26) und der Längsachse des Handgriffes (20) insbesondere zwischen 60° und 90° beträgt.

4. Lichtpolymerisationsgerät nach einem der vorhergehenden Ansprüche, **dadurch gekennzeichnet, dass** ein Kühlkörper (40) vorgesehen ist, der in direkter oder in indirekter Wärmeleitverbindung mit der Lichtquelle (14) steht und von dem Gebläse (26) mit Strömungsluft beaufschlagbar ist.

5. Lichtpolymerisationsgerät nach einem der vorhergehenden Ansprüche, **dadurch gekennzeichnet, dass** das Gebläse (26) in einem Strömungskanal angeordnet ist, der an Kühlrippen (46) eines Kühlkörpers (40) entlang verläuft, und dass das Gebläse (26) saugseitig und/oder druckseitig der Kühlrippen (46) angeordnet ist.

6. Lichtpolymerisationsgerät nach einem der vorhergehenden Ansprüche, **dadurch gekennzeichnet, dass** ein Sockel (42) der Lichtquelle (14) und/oder die Lichtquelle (14) in den Strömungskanal hineinragt.

7. Lichtpolymerisationsgerät nach einem der vorhergehenden Ansprüche, **dadurch gekennzeichnet, dass** der Kühlkörper (40) im Wesentlichen zylindrisch ausgebildet ist und insbesondere seine Kühlrippen (46) sich im Wesentlichen parallel zur Drehachse des Gebläses (26) erstrecken.

8. Lichtpolymerisationsgerät nach einem der vorhergehenden Ansprüche, **dadurch gekennzeichnet, dass** das Gehäuse (12) im Bereich des Gebläses an zwei einander im Wesentlichen gegenüberliegenden Seiten mit mindestens einer Lufteintrittsöffnung und mindestens einer Luftaustrittsöffnung (28) versehen ist, die Luftschlitze (30) aufweisen, zwischen denen Stege vorgesehen sind, wobei insbesondere die Stege im Wesentlichen in der Verlängerung der Kühlrippen (46) angeordnet sind.

9. Lichtpolymerisationsgerät nach einem der vorhergehenden Ansprüche, **dadurch gekennzeichnet, dass** das Gebläse (26) mindestens teilweise in einer Ausnehmung des Kühlkörpers (40) aufgenommen ist und insbesondere stirnseitige Endbereiche des Kühlkörpers (40) ausgebildet sind, zwischen denen das Gebläse (26) aufgenommen ist.

10. Lichtpolymerisationsgerät nach einem der vorhergehenden Ansprüche, **dadurch gekennzeichnet, dass** die Luftaustrittsöffnung (28) oder die Lufteintrittsöffnung in einem Gehäuseteil (32) angeordnet sind, das lösbar mit dem restlichen Gehäuse (12) in Verbindung steht.

11. Lichtpolymerisationsgerät nach Anspruch 10, **dadurch gekennzeichnet, dass** ein Auslöseelement, insbesondere ein Druckknopf (34), an dem Gehäuse (12) angeordnet ist, mit welchem die Verbindung zwischen dem lösbaren Gehäuseteil (32) und dem Gehäuse (12) im Übrigen freigebbar ist.

12. Lichtpolymerisationsgerät nach Anspruch 10 oder 11, **dadurch gekennzeichnet, dass** bei abgenommenem Gehäuseteil (32) das Gebläse (26, 27) aus dem Gehäuse entfernbar ist.

13. Lichtpolymerisationsgerät nach einem der vorhergehenden Ansprüche, **dadurch gekennzeichnet, dass** entlang der optischen Achse der Lichtquelle (14) oder entlang der Längsachse des Handgriffs (20) eine Mehrzahl von Gebläsen (26) hintereinander angeordnet sind, deren Drehachsen im Wesentlichen zueinander parallel verlaufen und ein ein- oder mehrteilige Kühlkörpers (40) sich von der Lichtquelle (14) und/oder deren Sockel (42) zu den Strömungskanälen der Gebläse (26) erstreckt und Kühlrippen (46) sich bezogen auf die optische Achse (76) quer erstrecken.

## Claims

1. A photopolymerisation device, in particular for curing photopolymerisable dental materials, with a hand grip (20), a housing and a fan (26) installed in the housing, in particular an axial fan, and a light source which emits polymerising radiation, **characterised in that** the axis of rotation of the fan (26) extends both transversely to the optical axis (76) of the light source (14) and transversely to the longitudinal axis of the hand grip (20).

2. A photopolymerisation device according to Claim 1, **characterised in that** the axis of rotation is arranged at an angle of 60° to 90° to the optical axis (76).

3. A photopolymerisation device according to one of the preceding Claims, **characterised in that** the angle between the axis of rotation of the fan (26) and the longitudinal axis of the hand grip (20) is in particular between 60° and 90°.

4. A photopolymerisation device according to any one of the preceding Claims, **characterised in that** a cooling body (40) is provided which is in direct or indirect heat-conducting connection with the light source (14) and can be acted upon with an air flow from the fan (26).

5. A photopolymerisation device according to any one of the preceding Claims, **characterised in that** the fan (26) is arranged in a flow duct which extends along cooling fins (46) of a cooling body (40), and **in that** the fan (26) is arranged on the intake side,and/or delivery side of the cooling fins (46).

6. A photopolymerisation device according to any one of the preceding Claims, **characterised in that** a base (42) of the light source (14) and/or the light source (14) projects into the flow duct.

7. A photopolymerisation device according to any one of the preceding Claims, **characterised in that** the cooling body (40) is of substantially cylindrical form and in particular its cooling fins (46) extend substantially parallel to the axis of rotation of the fan (26).

8. A photopolymerisation device according to any one of the preceding Claims, **characterised in that** in the vicinity of the fan on two substantially opposite sides the housing (12) is provided with at least one air-inlet opening and at least one air-outlet opening (28), which have air slots (30) between which webs are provided, wherein in particular the webs are arranged substantially in the extension of the cooling fins (46).

9. A photopolymerisation device according to any one of the preceding Claims, **characterised in that** the fan (26) is accommodated at least partly in a recess of the cooling body (40) and, in particular, end face end zones of the cooling body (40) are formed, between which the fan (26) is accommodated.

10. A photopolymerisation device according to any one of the preceding Claims, **characterised in that** the air-outlet opening (28) or the air-inlet opening is arranged in a housing part (32) which is releasably connected to the rest of the housing (12).

11. A photopolymerisation device according to Claim 10, **characterised in that** a release element, in particular a pushbutton (34), is arranged on the housing (12), with which it is possible to loosen, moreover, the connection between the releasable housing part (32) and the housing (12).

12. A photopolymerisation device according to Claim 10 or 11, **characterised in that** with the housing part (32) detached the fan (26,27) can be removed from the housing.

13. A photopolymerisation device according to any one of the preceding Claims, in that a plurality of fans (26) are in succession along the optical axis of the light source (14) or along the longitudinal axis of the hand grip (20), the axes of rotation of which fans extend substantially parallel to one another and a one-piece or multipart cooling body (40) extends from the light source (14) and/or its base (42) towards the flow ducts of the fan (26), and cooling fins (46) extend transversely in relation to the optical axis (76).

## Revendications

1. Appareil de photopolymérisation, en particulier pour durcir des matériaux dentaires photopolymérisables, avec une poignée (20), un boîtier et une soufflante (26) intégrée dans le boîtier, en particulier une soufflante axiale, et une source lumineuse, émettant le rayonnement de polymérisation, **caractérisé en ce que** l'axe de rotation de la soufflante (26) s'étend, tant transversalement à l'axe optique (76) de la source lumineuse (14), qu'également transversalement à l'axe longitudinal de la poignée (20).

2. Appareil de photopolymérisation selon la revendication 1, **caractérisé en ce que** l'axe de rotation est disposé sous un angle de 60° à 90° par rapport à l'axe optique (76).

3. Appareil de photopolymérisation selon l'une des revendications précédentes, **caractérisé en ce que** l'angle entre l'axe de rotation de la soufflante (26) et l'axe longitudinal de la poignée (20), est d'une valeur en particulier comprise dans la fourchette entre 60° et 90°.

4. Appareil de photopolymérisation selon l'une des revendications précédentes, **caractérisé en ce qu'**un corps de refroidissement (40) est prévu, placé en liaison de conduction thermique directe ou indirecte avec la source lumineuse (14) et susceptible d'être sollicité par de l'air en écoulement, venant de la soufflante (26).

5. Appareil de photopolymérisation selon l'une des revendications précédentes, **caractérisé en ce que** la soufflante (26) est disposée dans un canal d'écoulement, s'étendant le long d'ailettes de refroidissement (46) d'un corps de refroidissement (40), et **en ce que** la soufflante (26) est disposée côté aspiration et/ou refoulement des ailettes de refroidissement (46).

6. Appareil de photopolymérisation selon l'une des revendications précédentes, **caractérisé en ce qu'**un socle (42) de la source lumineuse (14) et/ou la source lumineuse (14) pénètre dans le canal d'écoulement.

7. Appareil de photopolymérisation selon l'une des revendications précédentes, **caractérisé en ce que** le corps de refroidissement (40) est réalisé sensiblement cylindrique, et en particulier ses ailettes de refroidissement (46) s'étendent sensiblement parallèlement à l'axe de rotation de la soufflante (26).

8. Appareil de photopolymérisation selon l'une des revendications précédentes, **caractérisé en ce que** le boîtier (12) est muni, dans la zone de la soufflante, sur deux côtés sensiblement opposés l'un à l'autre, d'au moins une ouverture d'entrée d'air, et d'au moins une ouverture de sortie d'air (28), qui présentent des fentes de passage d'air (30) entre lesquelles sont prévues des nervures, sachant qu'en particulier les nervures sont disposées sensiblement dans le prolongement des ailettes de refroidissement (46).

9. Appareil de photopolymérisation selon l'une des revendications précédentes, **caractérisé en ce que** la soufflante (26) est logée au moins partiellement dans un évidement du corps de refroidissement (40) et en particulier sont réalisées des zones d'extrémité, situées côté frontal, du corps de refroidissement (40), entre lesquelles la soufflante (26) est logée.

10. Appareil de photopolymérisation selon l'une des revendications précédentes, **caractérisé en que** l'ouverture de sortie d'air (28) ou l'ouverture d'entrée d'air est disposée dans une partie de boîtier (32), placée en liaison désolidarisable avec le reste du boîtier (12).

11. Appareil de photopolymérisation selon la revendication 10, **caractérisé en ce qu'**un élément de déclenchement, en particulier un bouton poussoir (34), est disposé sur le boîtier (12), bouton à l'aide duquel la liaison entre la partie de boîtier (32) désolidarisable et le boîtier (12) est au reste susceptible d'être coupée.

12. Appareil de photopolymérisation selon la revendication 10 ou 11, **caractérisé en ce que** la soufflante (26, 27) est susceptible d'être enlevée du boîtier lorsque la partie de boîtier (32) est démontée.

13. Appareil de photopolymérisation selon l'une des revendications précédentes, **caractérisé en ce que** le long de l'axe optique de la source lumineuse (14) ou le long de l'axe longitudinal de la poignée (20), sont disposées l'une dernière l'autre une pluralité de soufflantes (26), dont les axes de rotation s'étendent sensiblement parallèlement les uns aux autres, et un corps de refroidissement (40), en une ou plusieurs parties, s'étend de la source lumineuse (14) et/ou de son socle (42) vers les canaux d'écoulement des soufflantes (26), et des ailettes de refroidissement (46) s'étendent transversalement à l'axe optique (76).
